# EUROPEAN PATENT SPECIFICATION

(11) **EP 0 481 394 B1**
(45) Date of publication and mention of the grant of the patent: **06.09.1995**
(21) Application number: 91117468.8
(22) Date of filing: 14.10.1991
(51) Int. Cl.: C07C 255/25

(54) **Preparation of aminonitriles**
Herstellung von Aminonitrilen
Préparation d'aminonitriles

(30) Priority: 15.10.1990 US 597625
(43) Date of publication of application: 22.04.1992
(73) Proprietor: THE DOW CHEMICAL COMPANY, Midland, Michigan 48640 (US)
(72) Inventor: Crump, Druce K., Lake Jackson, Texas 77566 (US); Wilson, David A., Richwood, Texas 77531 (US)
(74) Representative: Huber, Bernhard, Dipl.-Chem.

(56) References cited:
- EP-A- 0 085 277
- US-A- 2 860 164

## Description

This invention relates to the preparation of aminonitriles.

Aminonitriles are useful, for instance in preparing aminocarboxylic acid compounds. For example, ethylenediaminetetraacetonitrile is hydrolyzed to prepare ethylenediaminetetraacetic acid (EDTA). Processes for the hydrolysis are known in the art such as are exemplified by the teachings of U.S. Patents 2,407,645; 2,164,781; and 2,205,995.

Aminonitriles also known as aminocarboxylic acid nitriles, have been prepared in a number of ways, often from certain amines reacted with certain carbonyl compounds particularly formaldehyde, and hydrocyanic acid (hydrogen cyanide, HCN). For instance, in U.S. Patent 2,205,995 (Ulrich, et al.), a process involves a reaction of certain amine salts with certain carbonyl compounds and hydrocyanic acid prepared from acidified cyanide salts. Ethylenediaminetetraacetonitrile is among the compounds prepared. Similarly, in U.S. Patent 2,407,645, Bersworth discloses a process for preparing certain polycarboxylic amino acids from certain aliphatic amines reacted with formaldehyde and an alkali metal cyanide. In U.S. Patent 4,855,428, Singer teaches yet another process in which certain amines are fed into a reaction medium containing formaldehyde and hydrocyanic acid. The medium is acidified and remains liquid. In U.S. Patent 3,424,783 Harper et al. teach reacting an amine with formaldehyde and hydrocyanic acid in the presence of an aqueous slurry of acidic ionic exchange resin to produce certain aminonitriles. In U.S. Patents 3,463,805 and 3,515,742 Morgan et al. emphasize characteristics of adiabatic conditions in reacting certain amines with formaldehyde and hydrocyanic acid in the presence of certain acidic catalyst. More recently, in U.S. Patent 4,478,759, Distler et al. disclose a process for reacting certain nitrogen compounds with formaldehyde and hydrocyanic acid in the presence of additional acids such that the pH is less than 2 and temperatures are from 10°C to 70°C. The concentration of hydrocyanic acid is controlled carefully. In U.S. Patent 4,704,465 Lannert et al. disclose a process for combining formaldehyde with ethylenediamine under certain conditions. A two-stage process is taught in U.S. Patent 4,560,516 (Singer). Other disclosures of reactions of certain amines with formaldehyde and hydrocyanic acid include, U.S. Patents 3,644,444 (Popper et al.); 3,679,729 (Daniels); 3,714,223 (Godfrey et al.); 3,758,534 (Popper et al.); and 3,988,360 (Gaudette et al.). Additionally, formaldehyde and hydrocyanic acid are reacted with ammonia to produce nitrilotriacetonitrile by processes such as those disclosed in U.S. Patents 3,907,858 (Davis et al.); 3,925,448 (Lanier) and 3,959,342 (Homberg et al.).

In some instances glycolonitrile (also known as glyconitrile and hydroxyacetonitrile) has been reacted with amines. For instance, in U.S. Patent 2,860,164 Kroll discloses certain carboxymethylations of certain amines by reacting glycolonitrile with primary or secondary amines in an aqueous solution at temperatures greater than 85°C to the boiling point of the mixture in the presence of basic hydroxides of alkali metals or alkaline earth metals or quaternary ammonium hydroxide to achieve carboxymethylation of the amine. In Column 2, lines 48-51 of this patent, Kroll discloses previous reactions of glycolonitrile with amines where reactions at primary amines are limited to reaction of the first hydrogen on each nitrogen.

EP-A-0 085 277 discloses a process for the production of ethylenediamine tetraacetic acid, which comprises reacting EDA with formaldehyde to form an adduct which is then reacted with hydrocyanic acid to form ethylenediamine diacetonitrile. This product is afterwards reacted with formaldehyde and hydrocyanic acid to obtain ethylenediamine tetraacetonitrile.

The present invention is a process for preparing amino acetonitriles comprising the steps of (a) admixing glycolonitrile with an amine having at least one primary amine group to form a reaction product, (b) admixing the reaction product of Step (a) with formaldehyde and hydrocyanic acid such that each hydrogen on an amine nitrogen is replaced by an acetonitrile group.

Amines suitable for the process of the invention are amines having at least one primary amine group. The amines preferably have from 2 to 20 carbon atoms and preferably have at least 2 amine groups, more preferably from 2 to 4 amine groups. Of these amine groups, at least one is primary, preferably at least 2 are primary, most preferably 2 amine groups per molecule are primary amine groups.

Suitable amines include ethylenediamine (EDA), aminoethylethanolamine (AEEA), diethylenetriamine (DETA), methylamine, ethylamine, n-propylamine, isopropylamine, n-butylamine, isobutylamine, sec-butylamine, tert-butylamine, pentylamine, pentyl-2-amine, propylenediamine, isopropylenediamine, sec-butylenediamine, triethylenetetraamine, dipropylenetriamine, diisopropylenetriamine, tetraethylenepentaamine, dibutylenetriamine, diisobutylenetriamine, ditert-butylenetriamine, pentaethylenehexaamine, tripropylenetetramine. Such amines are commercially available or are prepared by processes within the skill in the art such as by 1) the reaction of ammonia with alkyl halides (with and without catalysts), 2) catalytic amination of alcohols or amino alcohols, or 3) catalytic reduction of nitriles.

The amines are unsubstituted or inertly substituted, that is, substituted with groups which do not undesirably interfere with the reaction steps of the invention. Such inert substitution includes, for instance, hydroxyalkyl groups, carboxylic acid groups, sulfonic acid groups, and phosphonic acid groups. However, substitution is generally not desirable since the solubility in water may be increased. Increased solubility impairs isolation of the nitrile product.

The amine is admixed with glycolonitrile which is commercially available and is obtained by the reaction of HCN with formaldehyde which is within the state of the art such as illustrated by U.S. Patents 2,731,490 and 2,890,238.

Preferred ratios of glycolonitrile to amine in the first step depend on the amine. For example, with ethylenediamine (EDA), the most preferred ratio is one mole of EDA to 1.9 to 2.0 moles of glycolonitrile. In the case of diethylenetriamine, 2.8 to 3.0 moles of glycolonitrile are most preferably used with one mole of the amine. In general, the amount of glycolonitrile used is preferably less than or equal to an equivalent amount based on primary and secondary amine nitrogens of the amine. A primary amino group will accept one glycolonitrile addition, but under the preferred conditions for the reaction a second acetonitrile group is not conveniently added using glycolonitrile. Glycolonitrile does add to secondary alkyl amines, such as the secondary amino group of diethylenetriamine. Thus the equivalent amount of glycolonitrile for an amine having one primary and one secondary amine is two equivalents of glycolonitrile. Quantities of glycolonitrile greater than the number of equivalents of primary and secondary amine groups are generally disadvantageous because color and impurities are observed. More preferably, the ratio is 0.95 to 0.99 percent of the theoretical equivalents that can be added, to avoid unwanted color.

The reaction of amine and glycolonitrile is suitably carried out at a pH sufficient to allow the glycolonitrile to react with the amine, that is a pH sufficiently high that the amine groups are not protonated, that is preferably a basic pH above 8, more preferably from 8 to 14, most preferably from 9 to 13, even more preferably from 10 to 12.5. The amine reactant is generally sufficiently basic to achieve the preferred pH in a reaction medium of amine and glycolonitrile. Alternatively, the pH is adjusted prior to or during the reaction using any basic material which does not interfere undesirably with the reaction, e.g. sodium hydroxide. Additional basic materials are generally disadvantageous in that such materials are advantageously neutralized for the reaction with hydrocyanic acid. When, however, the amine is insufficiently basic to result in a reaction mixture of the preferred basicity, such as when the amine has an acidic group, e.g. glycine, additional basic materials are advantageously used.

The reaction is suitably carried out under any conditions under which the amino hydrogens react with the glycolonitrile, batch wise or, advantageously, continuously. The temperature is preferably sufficient to maintain the reaction of the glycolonitrile with the amine at a desirable rate, more preferably from 0°C to 90°C, even more preferably from 5°C to 80°C, most preferably from 10°C to 60°C under reduced pressure, atmospheric pressure or superatmospheric pressure.

The reaction preferably is carried out in the presence of water, more preferably in aqueous solution. The total amount of water is suitably any amount which allows solubility of the reaction product of amine and glycolonitrile and which also does not interfere undesirably with subsequent reaction steps, preferably from 10 to 90, more preferably from 15 to 50 percent by weight water, based on the total weight of amine and glycolonitrile reaction mixture.

The reaction mixture for the reaction of the primary amine groups, thus, comprises amine, glycolonitrile and, preferably, water. A solvent for the amine and/or glycolonitrile other than water is optionally used instead of the water or with the water. Preferably the solvent is one which dissolves both amine and glycolonitrile such as methanol, ethanol, or tiler auphatic alcohol with four or fewer carbon atoms. Use of solvents other than water generally involve use of purification methods other than precipitation of a solid product because the product is often soluble in such solvents. The reaction is, thus, preferably carried out in the presence of water.

The glycolonitrile reacts primarily with any secondary amine group and with one hydrogen on each primary amino group to form a reaction product having at least one secondary amine hydrogen remaining available for subsequent reaction. When it is desired that the remaining hydrogens (on amino groups having an acetonitrile group from the first reaction) be reacted to form an additional acetonitrile group, that reaction is carried out by reaction of the remaining hydrogens present on the amine with hydrocyanic acid and formaldehyde to form a fully derivatized polycarboxylic acid nitrile.

Conveniently, an aqueous solution of glycolonitrile is admixed with an amine for a time sufficient for the reaction to reach a predetermined degree of completeness, which is more preferably complete reaction. Then, preferably, the resulting reaction mixture is acidified before addition of subsequent reactants.

The reaction mixture is preferably adjusted to a pH of less than 5, more preferably of from 0 to 3, most preferably from 0.5 to 1. This pH is maintained by addition of a non-volatile mineral acid such as sulfuric acid or phosphoric acid.

Then, hydrogens on secondary amine group(s) (resulting from addition of glycolonitrile to primary amine groups) are admixed with formaldehyde and hydrocyanic acid (hydrogen cyanide, HCN) under reaction conditions such that each hydrogen on an amine nitrogen is replaced by an acetonitrile group. Advantageously, formaldehyde and hydrocyanic acid are added to the reaction mixture simultaneously.

Formaldehyde is commercially available and is suitably used in any form such as a liquid or a solid and is preferably used in the form of an aqueous solution, preferably of a strength of from 20 to 60 percent by weight, more preferably from 35 to 50 percent by weight formaldehyde. The formaldehyde is preferably used in any amount that will essentially completely substitute the remaining amino hydrogens during the reaction with HCN under reaction conditions, preferably in a ratio of 0.95 to 1.2 moles of formaldehyde to moles of remaining reactive amino hydrogens on the partially reacted amine. These mole ratios are preferred because it is generally desirable to fully substitute the amino hydrogens with amino acetonitrile groups to form an insoluble product and to minimize the partially substituted amine products in the reaction mixture.

Hydrogen cyanide is commercially available and is suitably used as a gas or, advantageously, as a liquid (either as a solution or pressurized sufficiently to result in a liquid). It is preferably introduced as a concentrated aqueous liquid or in pure liquid form. Slow addition (approximately equal to the rate at which it is reacted under reaction conditions) is desirable to avoid excess free cyanide in the reaction mixture. Thus, the concentration of unreacted hydrogen cyanide in a reaction mixture is preferably from 0 to 2, more preferably from 0.01 to 1.5, most preferably from 0.01 to 1 percent by weight based on total weight of the reaction mixture. The hydrogen cyanide is preferably used in an equivalent ratio of from 0.80 to 1.2 based on equivalents of remaining amino hydrogens, more preferably an equivalent ratio of from 0.9 to 1.15, most preferably from 0.95 to 1.1. These ranges are preferred because full aminoacetonitrile substitution is desired, yet excess unreacted HCN in the reaction medium is not desirable.

Any reaction conditions under which the hydrocyanic acid and formaldehyde react with the remaining amine hydrogens of the reaction product of the glycolonitrile and amine are suitable. Preferably, the reaction is carried out from 0°C to 90°C, more preferably from 25°C to 80°C, most preferably from 30°C to 70°C under reduced pressure, atmospheric pressure or superatmospheric pressure, batch wise or, continuously. The reaction is preferably carried out in the presence of water, advantageously introduced from the reaction mixture of glycolonitrile with the amine; water is also introduced as aqueous formaldehyde solution and/or aqueous hydrogen cyanide. The total amount of water is preferably from 20 to 90, more preferably from 30 to 80, most preferably from 40 to 75 weight percent based on weight of the reaction medium in which the HCN and formaldehyde are added.

The reaction medium thus contains the reaction product of the amine/glycolonitrile reaction, formaldehyde, hydrogen cyanide, preferably water, and acid. Use of a solvent other than water is optional, although not preferred. Such optional solvents include methanol, ethanol, or other aliphatic alcohols with four or fewer carbon atoms. Water is preferred because most aminoacetonitrile products are at least partially soluble in the solvents, and such solubility impairs their isolation by precipitation. However, such solvents are suitable when other isolation methods within the skill in the art, such as evaporation of solvent, are used.

The following examples are offered to illustrate the present invention. All parts, percentages, and ratios are by weight unless stated otherwise. Examples of the invention (Ex) are designated numerically, while comparative samples (C.S.) are designated alphabetically.

### Comparative Sample A: REACTION OF HYDROGEN CYANIDE AND FORMALDEHYDE WITH ETHYLENE DIAMINE

Ethylenediamine (EDA), 0.623 grams (98 percent pure) (0.01 moles), was added to a 3-dram vial containing a magnetic stir bar. Water was added to make a total of 1.943 grams. The vial was capped with a septum, and a small balloon was used to prevent escape of gases from the reaction system and allow for pressure equalization. The contents of the vial were continuously stirred and heated at 60°C. Then 1.63 grams 37 percent by weight formaldehyde (0.02 moles) and 0.63 grams 90 percent by weight HCN in water (0.021 moles) were added simultaneously over a 2 hour period via syringes using a syringe pump. The pH was adjusted to 5.4 with sulfuric acid and an additional 1.62 grams 37 percent formaldehyde and 0.64 grams of 90 percent HCN were added over a two hour period at 60°C. During the addition of the second half of the HCN and formaldehyde, a precipitate was observed. The quantity of precipitate increases as the addition continues. After the addition was complete, the heat was maintained for another 2 hours, after which the stirring was continued at room temperature for 12 hours. The precipitate (solids) was filtered, washed with a minimum amount of water, dried and weighed. Yield of ethylenediamine tetraacetonitrile (EDTN) (identified by melting point, hydrogen Nuclear Magnetic Resonance (¹H NMR), and Carbon 13 Nuclear Magnetic Resonance (¹³C NMR)) based on moles of EDA was 26 percent. The product was dissolved in hexadevtero methyl sulfoxide. The standard for ¹H NMR was Dioxane, and peaks at 2.71 ppm and 3.83 ppm from that standard were indicative of EDTN. The standard for ¹³C NMR was Dioxane and peaks at 42.1, 49.2 and 115.9 ppm from that standard were indicative of EDTN.

### Example 1: REACTION OF GLYCOLONITRILE WITH EDA AND SUBSEQUENT REACTION WITH FORMALDEHYDE AND HYDROCYANIC ACID

In an apparatus as used in Comparative Sample A, 2.85 grams of 40 percent by weight aqueous glycolonitrile (0.02 moles) was added over 2 hours to 0.01 moles EDA in water at 60°C while stirring. The pH was adjusted to 5.4 using sulfuric acid. A sample of 1.63 grams of 37 percent formaldehyde (0.02 moles) and 0.61 grams of 90 percent HCN (0.02 moles) were added simultaneously over 2 hours at 60°C. Early into the addition of the HCN and formaldehyde, a precipitate was observed which increased as the addition continued. Heat was continued for 2 hours after the addition was complete and stirring was continued at room temperature (25°C) for 12 more hours. The precipitate (solids) was filtered, washed, dried and weighed. Yield to EDTN (identified as in Comparative Sample A) based on EDA was 74 percent.

### Comparative Sample B: REACTION OF Glycolonitrile WITH ETHYLENEDIAMINE

The process of Example 1 was repeated using 2.85 grams of 40 percent glycolonitrile and 0.61 grams of EDA in water at 60°C added over a 2 hour period. Following adjustment of pH to 5.4, another 2.85 grams of 40 percent glycolonitrile was added over a 2 hour period at 60°C. Heat was continued for an additional 2 hours and stirring was maintained at room temperature for an additional 12 hours beyond the heating. Solids of EDTN were isolated as in Example 1 in 13 percent yield. (Solids were not observed until after stirring for some time at room temperature.)

EDTN, C₁₀H₁₂N₆, showed three types of carbons by ¹³C NMR, and two types of protons by ¹H NMR as described in Comparative Sample A. Melting point was 129°C.

Comparison of the yields of EDTN in Example 1 (74 percent), and Comparative Samples A (26 percent) and B (13 percent) show that a much greater yield was obtained when glycolonitrile was reacted with a primary amine, and the resulting product was reacted with formaldehyde and HCN under acidic conditions in contrast to the amine reaction with either formaldehyde and HCN alone or glycolonitrile alone.

## Claims

1. In a process for preparing aminoacetonitriles including a step of (a) admixing glycolonitrile with an amine having at least one primary amine group to form a reaction product, the improvement comprising in step (a) using an amount of glycolonitrile less than or equal to an equivalent amount based on primary and secondary amine nitrogens of the amine; and (b) admixing the reaction product of Step (a) with formaldehyde and hydrocyanic acid such that each hydrogen on an amine nitrogen is replaced by an acetonitrile group.

2. The process of Claim 1 wherein the amine has from 2 to 20 carbon atoms and amine groups.

3. The process of Claim 2 wherein the amine has from 2 to 4 amine groups, 2 of which are primary.

4. The process of Claim 3 wherein the amine is ethylenediamine (EDA).

5. The process of Claim 1, 2, 3 or 4 wherein the ratio of equivalents of glycolonitrile to equivalents of amine is from 0.95 to 0.99, and wherein step (a) takes place at a pH of from 8 to 14, and step (b) takes place at a pH of from 0 to 3.

6. The process of Claim 1, 2, 3, 4 or 5 wherein step (a) takes place at a pH above 8 and step (b) takes place at a pH of less than 5.

7. The process of Claim 1, 2, 3, 4, 5 or 6 wherein step (a) takes place at a temperature of from 0°C to 90°C and step (b) takes place at a temperature of from 0° to 90°C.

8. The process of Claim 1, 2, 3, 4, 5, 6 or 7 wherein either or both of steps (a) and (b) take place in aqueous solution.

9. The process of Claim 1, 2, 3, 4, 5, 6, 7 or 8 wherein in step (b), the formaldehyde and hydrocyanic acid are added simultaneously.

10. The process of Claim 1, 2, 3, 4, 5, 6, 7, 8 or 9 wherein the hydrocyanic acid is added at a rate approximately equal to the rate at which is reacted.

## Patentansprüche

1. In einem Verfahren zum Herstellen von Aminoacetonitrilen, das einen Schritt des (a) Zumischens von Glykolnitril zu einem Amin, das mindestens eine primäre Amingruppe enthält, um ein Reaktionsprodukt zu bilden, umfaßt, die Verbesserung, umfassend in Schritt (a) das Verwenden einer Menge von Glykolnitril, die, basierend auf primären oder sekundären Aminstickstoffen des Amins kleiner oder gleich einer äquivalenten Menge ist und (b) Zumischen des Reaktionsprodukts von Schritt (a) zu Formaldehyd und Cyanwasserstoff, derart, daß jeder Wasserstoff an einem Aminstickstoff durch eine Acetonitrilgruppe ersetzt wird.

2. Verfahren nach Anspruch 1, worin das Amin 2 bis 20 Kohlenstoffatome und Amingruppen enthält.

3. Verfahren nach Anspruch 2, worin das Amin 2 bis 4 Amingruppen enthält, von welchen 2 primär sind.

4. Verfahren nach Anspruch 3, worin das Amin Ethylendiamin (EDA) ist.

5. Verfahren nach Anspruch 1, 2, 3 oder 4, worin das Verhältnis von Glykolnitriläquivalenten zu Aminäquivalenten von 0,95 bis 0,99 ist und worin Schritt (a) bei einem pH von 8 bis 14 stattfindet und Schritt (b) bei einem pH von 0 bis 3 stattfindet.

6. Verfahren nach Anspruch 1, 2, 3, 4 oder 5, worin Schritt (a) bei einem pH über 8 stattfindet und Schritt (b) bei einem pH von weniger als 5 stattfindet.

7. Verfahren nach Anspruch 1, 2, 3, 4, 5 oder 6, worin Schritt (a) bei einer Temperatur von 0 °C bis 90 °C stattfindet und Schritt (b) einer Temperatur von 0 °C bis 90 °C stattfindet.

8. Verfahren nach Anspruch 1, 2, 3, 4, 5, 6 oder 7, worin einer oder beide der Schritte (a) und (b) in wäßriger Lösung stattfindet.

9. Verfahren von Anspruch 1, 2, 3, 4, 5, 6, 7 oder 8, worin in Schritt (b) das Formaldehyd und die Cyanwasserstoffsäure gleichzeitig zugegeben werden.

10. Verfahren von Anspruch 1, 2, 3, 4, 5, 6, 7, 8 oder 9, worin die Cyanwasserstoffsäure mit einer Geschwindigkeit zugegeben wird, die etwa gleich der Umsatzgeschwindigkeit ist.

## Revendications

1. Dans un procédé de préparation d'aminoacétonitriles comprenant une étape (a) de mélange de glycolonitrile avec une amine présentant au moins un groupe amine primaire pour former un produit de réaction, le perfectionnement qui comprend l'utilisation dans l'étape (a) d'une quantité de glycolonitrile inférieure ou égale à une quantité équivalente sur la base des azotes amine primaire et secondaire de l'amine ; et (b) le mélange du produit de réaction de l'étape (a) avec du formaldéhyde et de l'acide cyanhydrique en quantité telle que chaque hydrogène sur un azote d'amine soit remplacé par un groupe acétonitrile.

2. Procédé selon la revendication 1, dans lequel l'amine comporte 2 à 20 atomes de carbone et des groupes amine.

3. Procédé selon la revendication 2, dans lequel l'amine comporte de 2 à 4 groupes amine, deux de ces groupes étant primaires.

4. Procédé selon la revendication 3, dans lequel l'amine est l'éthylène diamine (EDA).

5. Procédé selon l'une des revendications 1, 2, 3 ou 4, dans lequel le rapport entre les équivalents de glycolonitrile et les équivalents d'amine est compris entre 0,95 et 0,99, et dans lequel l'étape (a) a lieu à un pH compris entre 8 et 14 et l'étape (b) a lieu à un pH compris entre 0 et 3.

6. Procédé selon l'une des revendications 1, 2, 3, 4 ou 5, dans lequel l'étape (a) a lieu à un pH supérieur à 8 et l'étape (b) a lieu à un pH inférieur à 5.

7. Procédé selon l'une des revendications 1, 2, 3, 4, 5 ou 6, dans lequel l'étape (a) a lieu à une température comprise entre 0°C et 90°C et l'étape (b) a lieu à une température comprise entre 0° et 90°C.

8. Procédé selon l'une des revendications 1, 2, 3, 4, 5, 6 ou 7, dans lequel l'une des étapes (a) et (b) ou les deux ont lieu en solution aqueuse.

9. Procédé selon l'une des revendications 1, 2, 3, 4, 5, 6, 7 ou 8, dans lequel dans l'étape (b), le formaldéhyde et l'acide cyanhydrique sont ajoutés simultanément.

10. Procédé selon l'une des revendications 1, 2, 3, 4, 5, 6, 7, 8 ou 9, dans lequel l'acide cyanhydrique est ajouté à une cadence approximativement égale à celle à laquelle il réagit.
